# FASCICULE DE BREVET EUROPEEN

(11) **EP 0 618 821 B1**
(45) Date de publication et mention de la délivrance du brevet: **02.01.1997**
(21) Numéro de dépôt: 93911832.9
(22) Date de dépôt: 28.04.1993
(51) Int. Cl.: A61M 5/172

(54) **SYSTEME DE POMPE A PERFUSION AMBULATOIRE PROGRAMMABLE**
TRAGBARE PROGRAMMIERBARE INFUSIONSPUMPE
PROGRAMMABLE PORTABLE INFUSION PUMP SYSTEM

(30) Priorité: 29.04.1992 FR 9205310
(43) Date de publication de la demande: 12.10.1994
(73) Titulaire: CHRONOTEC S.A.R.L., F-06600 Antibes (FR)
(72) Inventeur: HAUSER, Jean-Luc, F-06600 Antibes (FR)
(74) Mandataire: Bonneau, Gérard
(86) Numéro de dépôt international: FR9300414
(87) Numéro de publication internationale: WO9321978

(56) Documents cités:
- EP-A- 0 002 775
- EP-A- 0 188 288
- WO-A-84/00493
- US-A- 4 756 706

## Description

### Domaine technique

L'invention concerne un système de pompe à perfusion ambulatoire programmable destiné à injecter automatiquement une substance médicamenteuse à un patient, du type comprenant un réservoir de la substance médicamenteuse, un bloc-pompe comportant un moteur et une pompe entraînée par le moteur pour injecter la substance médicamenteuse dans une ligne de perfusion reliée au corps du patient, et une unité de commande programmable pour commander la mise en route du moteur à des instants programmés à l'avance.

### Etat de la technique

Il est devenu de plus en plus courant de traiter certaines maladies par perfusion régulière d'une substance médicamenteuse par voie intraveineuse, intra-artérielle, intrarachidienne... Pour ce faire, il existe des systèmes de pompe à perfusion installés au pied du lit du patient et pouvant être programmés de façon à effectuer régulièrement une ou plusieurs perfusions simultanées sur le patient auquel est raccordée la pompe. Un système de ce type est décrit dans le document US-A.4.756.706. Malheureusement, ce système de pompe à perfusion n'est pas transportable par le patient, et il s'adresse généralement à des grands malades soignés exclusivement en milieu hospitalier.

Cependant, beaucoup de patients nécessitent des traitements par perfusion, bien qu'ils conservent leur autonomie. Pour ceux-ci et dans le but de ne pas les obliger à effectuer des séjours trop fréquents en milieu hospitalier pour pratiquer les perfusions, des systèmes ambulatoires ont été mis au point.

On dispose alors d'une ligne de perfusion qu'on relie au corps du patient pour pratiquer la perfusion lorsque nécessaire. Lorsque la perfusion doit être pratiquée, un système de pompe à perfusion ambulatoire que le patient porte sur lui est mis en communication avec l'aiguille à perfusion. La perfusion est effectuée par une pompe entraînée par un moteur, pompe qui injecte la substance médicamenteuse provenant d'un réservoir dans le corps du patient par l'intermédiaire de la ligne de perfusion. Le moteur, la pompe et le réservoir sont intégrés dans le système de pompe à perfusion ambulatoire.

Des systèmes de pompe à perfusion ambulatoires programmables ont été mis au point. Dans un système de ce type, une unité de commande intégrée au système permet de programmer le protocole thérapeutique c'est-à-dire le dosage de la perfusion à effectuer, la durée de la perfusion et le moment auquel doit être déclenchée la perfusion. Les systèmes actuels de ce type comprennent à cet effet une unité de commande qui est en réalité un simple programmateur en ce sens qu'elle permet la programmation du protocole thérapeutique relativement à l'instant où le système est mis en marche, mais ne permet pas une programmation en fonction de l'heure solaire. En outre la programmation se fait généralement au moyen de touches multifonctions, ce qui limite les possibilités dans le cas où il y a peu de touches, ou augmente les possibilités d'erreurs dans le cas où il y a trop de fonctions.

Dans la majorité des systèmes actuels, seul le réservoir contenant la substance médicamenteuse, ou "cassette", est détachable de l'ensemble, ceci dans le but de pouvoir le remplacer lorsqu'il est vide. Par contre, le bloc-pompe et l'unité de commande ne forment qu'un seul élément, ce qui constitue un inconvénient majeur des systèmes de pompe à perfusion programmables actuels.

Il existe également des systèmes ambulatoires programmables tel que le système à perfusion d'insuline décrit dans le brevet US-A-4.270.532 dans lequel le bloc comprenant le moteur, la pompe et le réservoir d'insuline est détachable de l'unité de commande . Mais ce système présente deux inconvénients majeurs. En effet, il est de plus en plus courant d'administrer plusieurs substances médicamenteuses simultanément pour améliorer l'efficacité d'un traitement thérapeutique. Dans ce cas il faut donc que le patient se procure un deuxième système de pompe à perfusion ambulatoire pour pouvoir effectuer les deux perfusions simultanément tout en gardant son autonomie et sans être immobilisé en milieu hospitalier. Le coût de deux systèmes de pompe à perfusion ambulatoires devient alors prohibitif, sans parler de la difficulté pour le patient de transporter sur lui deux systèmes de pompe à perfusion qui, sans être lourds, n'en sont pas moins encombrants.

Un système de pompe à perfusion programmable pouvant effectuer plusieurs perfusions simultanément est décrit dans le brevet EP-A-0.188.288. Mais ce système présente un inconvénient majeur constituant le deuxième inconvénient du système décrit dans US-A-4.270.532. C'est que l'unité de commande n'est autonome qu'après avoir été programmée par un dispositif de programmation extérieur qui se connecte à l'unité de commande pour procéder à la programmation. Cette programmation nécessite donc que le patient se rende dans un milieu hospitalier où se trouve le dispositif de programmation chaque fois qu'il doit pratiquer une perfusion.

### Exposé de l'invention

Le but de la présente invention est donc de pouvoir réaliser deux ou plusieurs perfusions de façon ambulatoire simultanément en utilisant un système simple, peu encombrant et à un coût pas très élevé.

Un autre but de l'invention est de fournir un système de pompe à perfusion ambulatoire programmable ne nécessitant qu'une seule unité de commande pour pratiquer plusieurs perfusions simultanément.

Encore un autre but de l'invention est de fournir un système de pompe à perfusion ambulatoire programmable dans lequel la programmation se fait directement sur le patient grâce à une unité de commande programmable sans dispositif de programmation extérieur.

L'objet de l'invention est donc un système de pompe à perfusion ambulatoire programmable dans lequel l'unité de commande comprend un microprocesseur, un clavier et un écran, le microprocesseur étant programmé directement à l'aide du clavier pour commander le fonctionnement du moteur selon un programme de perfusion prédéterminé et fournissant des informations sur la perfusion par affichage sur l'écran.

Un autre objet de l'invention est un système de pompe à perfusion ambulatoire programmable du type ci-dessus comprenant en outre au moins un deuxième ensemble avec un deuxième bloc-pompe et un deuxième réservoir, connecté à l'unité de commande, le deuxième ensemble étant détachable de l'unité de commande tout en maintenant le deuxième bloc-pompe sous la commande de celle-ci, de sorte que l'unité de commande peut commander plusieurs perfusions simultanément.

En résumé, le système de pompe programmable selon la présente invention se caractérise par une grande modularité et une complète autonomie permettant au patient qui en est muni de ne pas être tributaire du milieu hospitalier ou d'être gêné dans sa vie courante.

### Brève description

Les buts, objets et caractéristiques de l'invention seront mieux compris à la lecture de la description suivante faite en référence aux dessins dans lesquels :
la figure 1 représente un schéma d'un mode de réalisation du système de pompe à perfusion ambulatoire programmable selon l'invention,
la figure 2 représente le système de pompe à perfusion ambulatoire illustré sur la figure 1 avec le bloc-pompe détaché de l'unité de commande,
la figure 3 représente un mode de réalisation de l'invention comportant une seule unité de commande utilisée pour la commande d'un bloc-pompe accolé et d'un bloc-pompe détaché,
la figure 4 représente un mode de réalisation du dispositif d'assemblage de la cassette sur le bloc-pompe,
la figure 4a est une vue en coupe du clips utilisé comme dispositif d'assemblage de la cassette sur le bloc-pompe illustré sur la figure 4,
la figure 4b représente le dispositif d'assemblage de la figure 4a en position ouverte,
la figure 5 est un schéma synoptique représentant les différents éléments composant le système programmable selon l'invention et leurs interconnexions,
la figure 6 est un organigramme représentant les étapes de configuration du système programmable selon l'invention
la figure 7 est un organigramme représentant les étapes de programmation du système programmable selon l'invention,
la figure 8 est un organigramme représentant les étapes de mise en marche du système programmable selon l'invention
la figure 9 est un organigramme représentant le fonctionnement simultané des deux blocs-pompes incorporés dans le système de perfusion programmable selon le mode de réalisation préféré de l'invention, et
les figures 10a-10d représentent l'unité de commande du système programmable selon l'invention aux différentes étapes de la procédure de programmation.

### Description détaillée

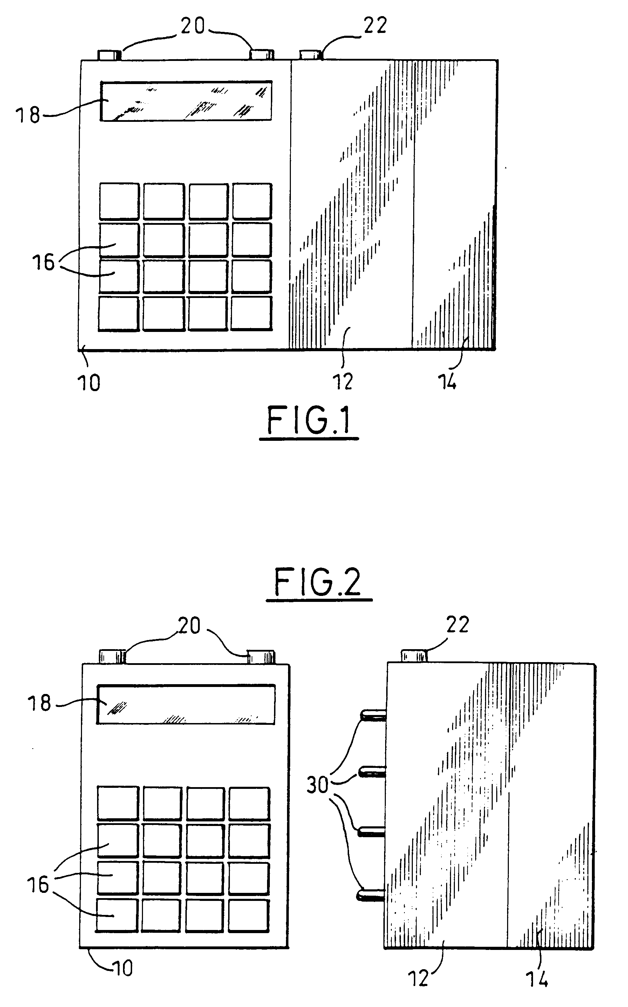

Comme illustré sur la figure 1, le système de pompe à perfusion ambulatoire programmable selon un mode de réalisation préféré de l'invention comprend une unité de commande 10, un bloc-pompe 12 et un réservoir 14, le tout formant un ensemble unique, compact, et facilement portatif pour le patient. L'unité de commande comprend un clavier 16 adapté pour entrer les commandes et en particulier les paramètres permettant de définir le protocole thérapeutique, et un écran 18, de préférence à cristaux liquides pour pouvoir afficher certains paramètres ou résultats de contrôle de la perfusion. Cette unité de commande peut être réalisée à l'aide d'un microprocesseur perfectionné et adapté spécifiquement pour disposer d'entrées/sorties adéquates. L'unité de commande comporte également deux bornes 20 utilisées pour la commande à distance de l'ensemble bloc-pompe et du réservoir détaché. L'unité de commande dispose de piles pour l'alimentation en énergie de tout le système, d'une unité de traitement, d'une mémoire morte et d'une mémoire vive.

Le bloc-pompe 12 comporte les mêmes éléments que ceux des systèmes de la technique antérieure, à savoir un moteur électrique commandé par l'unité de commande, une pompe du type pompe péristaltique ou tout autre pompe équivalente, un circuit électronique qui est relié à l'unité de commande, et la partie d'une ligne de perfusion en provenance du réservoir 14 qui subit l'action de la pompe pour provoquer l'écoulement de la substance médicamenteuse vers le corps du patient. Enfin le bloc-pompe comporte une borne 22 destinée à être connectée à l'unité de commande lorsque le bloc-pompe est détaché de l'unité de commande 10.

Le réservoir 14 est généralement constitué par une "cassette" à usage unique composée d'un sachet souple en polymère contenant la substance médicamenteuse et enfermée dans un boîtier rigide.

La figure 2 représente l'ensemble du bloc-pompe et de la cassette détaché de l'unité de commande. Un ensemble de 4 broches 30 permet au bloc-pompe 12 d'être connecté de façon solidaire à l'unité de commande. Mais les broches 40, outre leur rôle mécanique, ont également pour fonction d'assurer la liaison électrique entre l'unité de commande et le bloc-pompe lorsque ce dernier n'est pas détaché. Il est clair que la fonction des broches 30 pourrait être simplement électrique, la connexion mécanique étant alors effectuée par un moyen d'assemblage classique. Quand le bloc-pompe est détaché, soit les bornes sont recouvertes par une plaque, soit elles sont conçues pour être escamotables à l'intérieur du bloc-pompe en les poussant à l'intérieur du bloc-pompe après les avoir fait tourner d'un quart de tour.

Bien que sur les figures 1 et 2, un seul bloc-pompe (et la cassette associée) ait été représenté, il est possible d'avoir un autre bloc-pompe (et la cassette associée) fixé de l'autre côté de l'unité de commande. C'est un des avantages du système de l'invention que l'unité de commande peut commander simultanément deux blocs-pompes lorsque deux perfusions de substances médicamenteuses différentes sont à pratiquer simultanément. Ainsi, le deuxième bloc-pompe serait attaché au côté gauche de l'unité de commande par un ensemble de broches identiques aux broches 30 illustrées sur la figure 2.

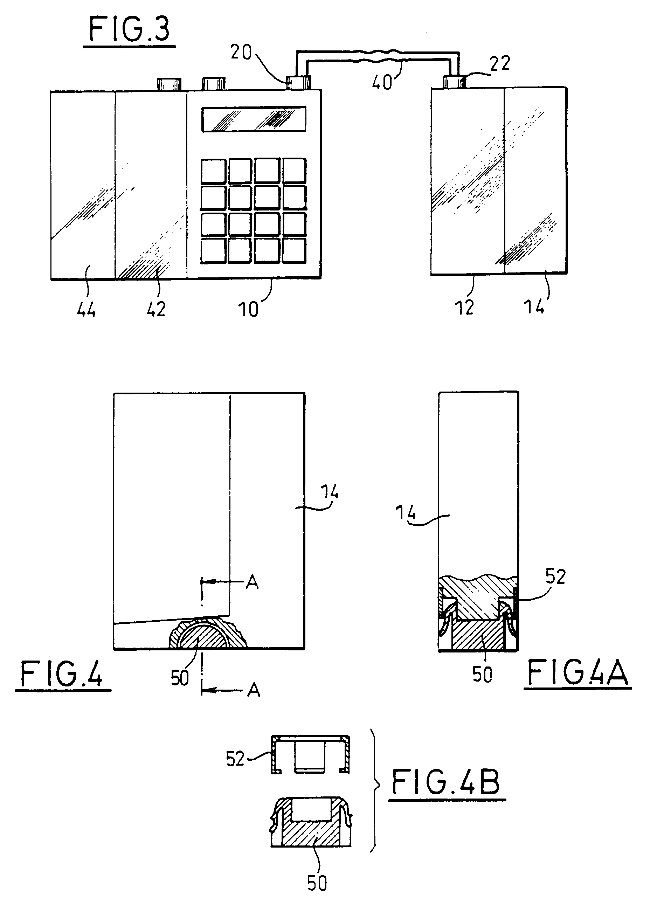

La commande par l'unité de commande de deux perfusions simultanées peut être réalisée autrement comme on va le voir sur la figure 3. Comme illustré, le bloc-pompe 12 et le réservoir associé 14 ont été détachés de l'unité de commande 10. Toutefois, une connexion électrique 40 relie l'unité de commande 10 au bloc-pompe 12 respectivement par les bornes 20 et 22, ce qui permet au bloc-pompe de rester sous la commande de l'unité de commande tout en étant placé à un endroit différent sur le patient. En même temps que l'unité de commande 10 garde la commande d'une perfusion pratiquée au moyen du bloc-pompe 12, la même unité de commande 10 a aussi la commande d'un deuxième bloc-pompe 42 et le réservoir associé 44 qui sont attachés directement à l'unité de commande 10.

On voit donc que, dans le mode de réalisation préféré de l'invention, l'unité de commande peut aussi commander quatre perfusions simultanées, en utilisant deux blocs-pompes directement attachés à l'unité de commande, et deux autres blocs-pompes connectés à distance au moyen des bornes 20 de l'unité de commande. Il est évident que le nombre de blocs-pompes pouvant être mis sous la commande de l'unité de commande n'est pas limité si ce n'est par la capacité de pratiquer un nombre maximal de perfusions simultanées.

La liaison amovible entre le bloc-pompe et le réservoir peut être réalisée selon un mode de réalisation illustré sur les figures 4 et 4a par un mécanisme de liaison à enclenchement élastique. Sur la figure 4, on voit que le réservoir comporte une partie horizontale disposant d'un clips 50 qui permet de verrouiller le réservoir ou "cassette" sur le bloc-pompe. La figure 4a illustre le clips de fermeture 50 selon la section A, en position fermée et en position ouverte, et montre bien comment deux équerres 52 solidaires du bloc-pompe viennent se bloquer sur deux épaulements du clips 50 solidaire du réservoir. Ce dispositif de verrouillage peut bien entendu être remplacé par tout autre dispositif de verrouillage sans sortir du cadre de l'invention.

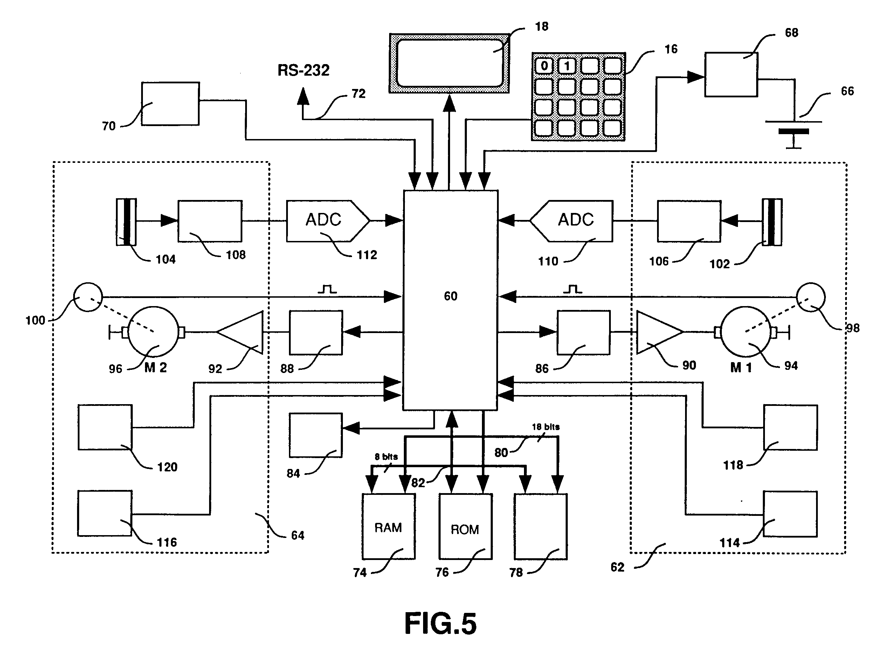

la figure 5 est un bloc-diagramme illustrant les divers éléments d'un système comportant deux blocs-pompes et leurs interconnexions

L'ensemble représenté sur la figure comprend un microprocesseur ou microcontrôleur qui constitue l'unité de traitement de l'unité de commande connectée à tous les éléments et en particulier aux deux blocs-pompes 62 et 64. Le microprocesseur dispose de son alimentation propre 66 (constituée par des piles) qui lui est connectée au moyen du circuit de surveillance de tension 68. Le circuit de surveillance 68 comporte un "chien de garde " de façon à interrompre le microprocesseur 60 en cas de défaillance de l'alimentation 66.

La programmation de l'unité de commande se fait principalement au moyen du clavier 16, mais peut se faire également par d'autres moyens connus. Ainsi la programmation peut se faire à l'aide d'un dispositif de lecture à codes barres et d'un stylet optique que le médecin ou l'infirmière n'a plus qu'à approcher du dispositif de lecture pour entrer le programme de commande d'une perfusion déterminée. Ou bien, on peut également concevoir la programmation de l'unité de commande à l'aide d'une carte à puce qu'il suffira d'introduire dans l'unité de commande ou dans un élément accessoire, qui lui est connecté pour immédiatement y entrer la programmation désirée. Le moyen préféré est un dispositif de lecture de codes à barres 70 représenté sur la figure 5.

L'unité de commande peut également être connectée à un ordinateur de bureau par une liaison 72 du type RS-232. Une telle connexion pourra permettre de programmer l'unité de commande à partir de l'ordinateur plutôt qu'à partir de son clavier, et également de stocker les données recueillies dans la mémoire de l'unité de commande à des fins statistiques. Cette même liaison peut d'ailleurs être utilisée pour le branchement d'un modem. De cette façon il sera aisé au patient de brancher son unité de commande sur le réseau téléphonique pour qu'un médecin disposant également d'un modem puisse exercer une télésurveillance du bon déroulement du traitement thérapeutique, ou même procède à la programmation de l'unité de commande au moyen d'instructions transmises par la ligne téléphonique.

Enfin, l'unité de commande pourrait également disposer d'un récepteur d'ondes radio pour pouvoir être programmée à distance à l'aide d'un émetteur correspondant, à condition qu'un code soit nécessaire pour qu'une telle programmation puisse avoir lieu.

L'affichage des informations recueillies au cours de la perfusion, ou des données introduites durant la programmation par le médecin à l'aide du clavier 16 ou du lecteur de code à barres 70 se fait à l'aide de l'écran 18 qui est de préférence un écran à cristaux liquides de 4 lignes et de 16 caractères.

L'unité de commande dispose d'une mémoire vive RAM 74 d'une capacité de 512 kilo-octets chargée de stocker les données entrées au clavier et les informations sur la perfusion, une mémoire morte ROM 76 contenant les instructions du programme de commande des opérations effectuées par le système, également d'une capacité de 512 kilo-octets, qui est de préférence une mémoire du type EPROM, c'est à dire électriquement effaçable et reprogrammable pour que le logiciel puisse être facilement mis à jour. L'unité de commande dispose bien entendu d'une horloge temps réel 78 indispensable pour faire de la chronothérapie c'est à dire programmer les perfusions à pratiquer (en particulier le débit d'écoulement de la substance et son évolution) en fonction du temps universel et non pas à des intervalles de temps déterminés relativement à l'instant de mise en marche du système. La mémoire RAM 74, la mémoire ROM 76 et l'horloge temps réel 78 sont adressées par le microprocesseur 60 au moyen d'un bus d'adressage 80 de 16 bits, et l'échange des données pour ces trois unités se fait au moyen d'un bus de données 82 de 8 bits.

Un dispositif d'alarme ou "beep" 84 peut être connecté au microprocesseur 60 de façon à émettre un signal sonore dans le cas d'une interruption ou défaillance du système pendant qu'une perfusion est en cours.

Chacun des deux blocs-pompes 62 ou 64 est connecté au microprocesseur 60 au moyen d'un circuit de commande 86 ou 88 muni d'un "chien de garde", qui transforme les ordres du microprocesseur 60 en signaux électriques de commande, et d'un amplificateur 90 ou 92. Chacun des moteurs 94 (M1) ou 96 (M2) dispose d'un codeur magnétique 98 ou 100 chargé de transmettre des tops codeurs au microprocesseur 60 dans un but de régulation de la vitesse du moteur.

La partie de la ligne de perfusion qui se trouve dans le bloc-pompe est munie d'un capteur de pression 102 ou 104 chargé de fournir la pression régnant dans cette partie de la ligne soumise à l'action de la pompe, au microprocesseur 60 après mise en forme par le circuit de mise en forme 106 ou 108 et conversion d'analogique en numérique au moyen du convertisseur analogique-numérique (ADC) 110 ou 112.

Cette partie de la ligne de perfusion se trouvant dans le bloc-pompe est également munie d'un détecteur de bulles d'air 114 ou 116 pour avertir l'unité de commande au cas ou des bulles d'air auraient été introduites dans la ligne de perfusion.

Enfin, chaque bloc-pompe 62 ou 64 dispose d'un détecteur de cassette et de bloc-pompe 118 ou 120 qui informe automatiquement le microprocesseur 60 du raccordement du bloc-pompe à l'unité de commande ou de l'installation d'une cassette.

Un tel système de pompe à perfusion ambulatoire présente de nombreux avantages par rapport à la technique antérieure. L'unité de commande peut ainsi être programmée pour faire de la chronothérapie, comme on l'a vu précédemment. Cette possibilité permet d'adapter la perfusion au rythme biologique du patient. De même l'unité de commande dispose d'une mémoire (RAM) dans laquelle sont stockées les données concernant le patient. On peut ainsi programmer le protocole en tenant compte de certains paramètres de contrôle stockées en mémoire qu'il ne faudra pas dépasser au cours de la perfusion. Il est également possible de relier l'unité de commande à des capteurs se trouvant dans ou sur le corps du patient et qui transmettent à l'unité de commande des données physiologiques permettant à cette dernière de commander les paramètres de perfusion en fonction des données reçues. La mémoire sert également à emmagasiner toutes les données, résultats et incidents éventuels de la perfusion, et ainsi de connaître l'historique de la thérapie effectuée.

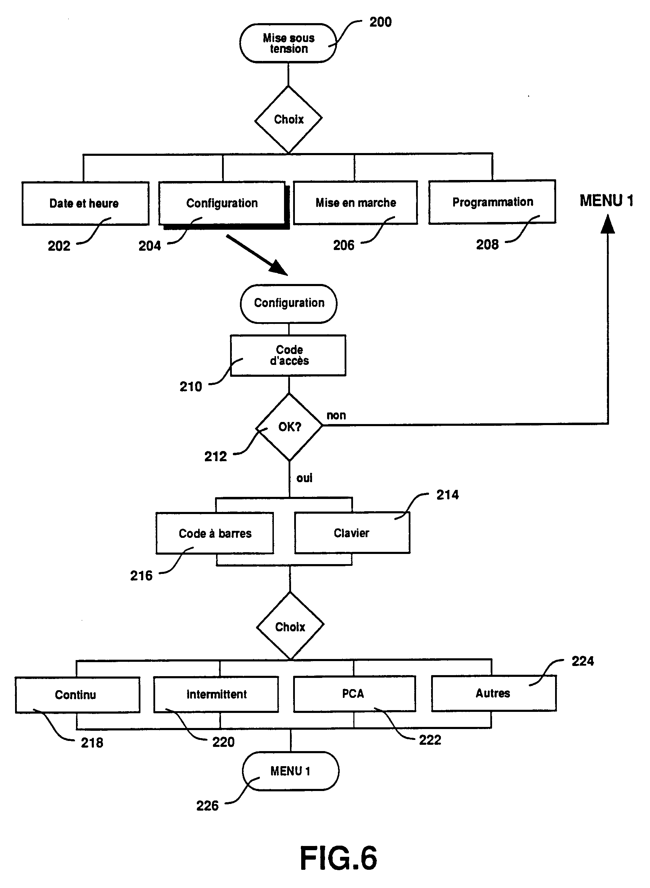

Les différentes procédures pouvant être mise en oeuvre avec le système selon l'invention vont maintenant être décrites en référence aux figures 6 à 9.

Comme illustré sur la figure 6, après la mise sous tension (200), un choix est présenté à l'opérateur entre plusieurs procédures possibles qui s'affichent sur l'écran 18 de l'unité de commande: DATE ET HEURE (202), CONFIGURATION (204), MISE EN MARCHE (206) et PROGRAMMATION (208)

La procédure DATE ET HEURE, bien qu'indispensable puisqu'elle permet la programmation de l'horloge temps réel de l'unité de commande, ne présente pas d'intérêt et ne sera pas décrite au détail.

Une des caractéristiques essentielles du système de pompe ambulatoire programmable de l'invention est de pouvoir être programmé par un médecin ou spécialiste directement sur le patient, mais également de faciliter son exploitation par le personnel soignant. Il est donc prévu de configurer le système dans un mode de fonctionnement déterminé préalablement à toute programmation. L'interface homme/machine est de celle façon grandement simplifié et l'utilisation du système devient plus conviviale.

La procédure CONFIGURATION est donc choisie au moyen d'un curseur déplacé à l'aide des touches du clavier. L'écran affiche alors "code d'accès" (210) invitant l'opérateur à entrer son code d'accès. Si un mauvais code d'accès est entré, l'opérateur est renvoyé (212) au menu 1 et les noms des procédures possibles s'affichent à nouveau. Si le code d'accès est correct, le choix est proposé d'entrer la configuration soit par le clavier (214) soit par le lecteur de code à barres (216). Après ce choix, l'écran affiche les différents modes possibles: MODE CONTINU (218), PCA (222) et AUTRES (224). Le mode PCA (Patient Control Analgesia) est un mode antidouleur effectué à l'aide de morphine ou autre analgésique.

Enfin, après l'entrée des données correspondant à un mode de configuration déterminé, le système retourne au menu 1 (226)

Une fois le système configuré dans un mode, le logiciel ne proposera que ce mode de configuration jusqu'à ce qu'une autre configuration soit entrée dans le système. L'avantage de cette procédure permet de modifier à volonté le parc de systèmes suivant les besoins. Les manipulations de programmation ultérieure sont réduites au strict minimum ce qui améliore grandement l'utilisation.

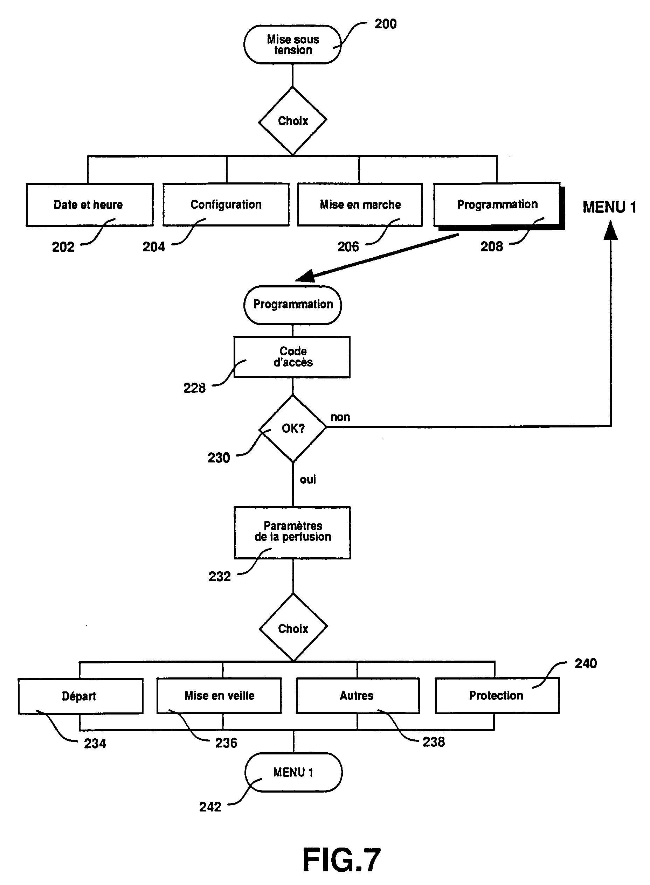

Après la configuration du système, la pompe peut être programmée pour une perfusion comme l'illustre la figure 7.

Lorsque la procédure PROGRAMMATION a été choisie (208) dans le menu 1, l'écran affiche la demande d'entrée du code d'accès (228). Si un mauvais code d'accès est entré, la décision (230) est de retourner au menu 1. Si le bon code d'accès est entré, l'unité de commande affiche les différents paramètres de la perfusion à entrer à l'aide du clavier (232). Après que les paramètres de la perfusion ont été entrés, un choix est proposé à l'opérateur entre le DEPART de la perfusion (234), MISE EN VEILLE (236), AUTRES (238) et protection (240). Ces fonctions seront expliquées plus en détail par la suite.

Lorsque la programmation est terminée, le système revient (242) au menu 1 comme précédemment.

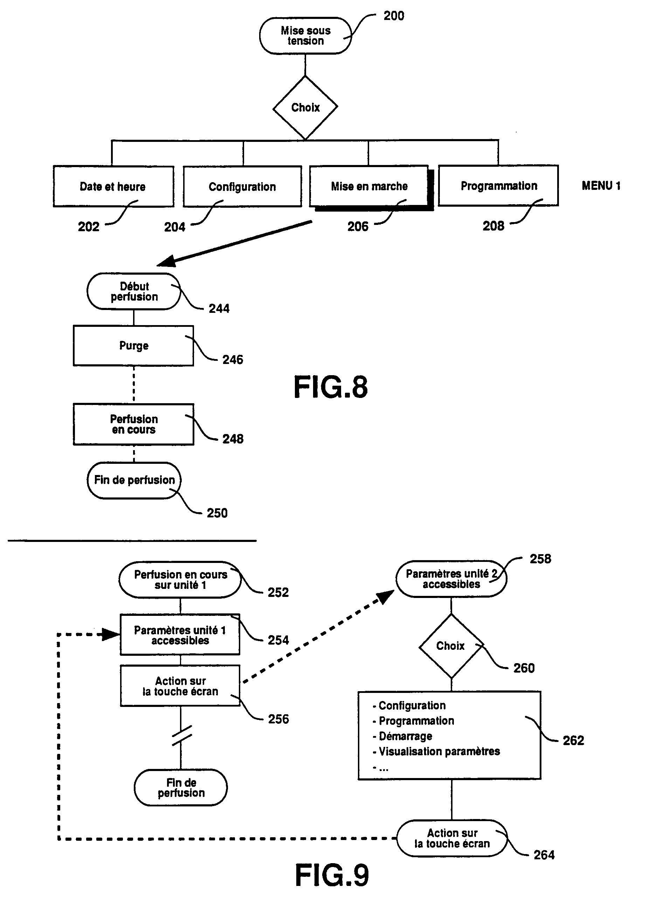

La figure 8 illustre la procédure de mise en marche (206) d'une perfusion. Après déclenchement du début de la perfusion (244), on procède à la purge de l'air (246) dans la ligne de perfusion. Puis la perfusion est opérée (248) avec la visualisation possible des données de la perfusion sur l'écran de l'unité de commande. La perfusion se termine par la fin de la perfusion (250) affichée sur l'écran.

Lorsque le système comporte deux blocs-pompes comme c'est le cas dans le mode de réalisation préféré de l'invention, l'enfoncement d'une touche spécifique du clavier permet d'accéder aux ressources du deuxième bloc-pompe. La deuxième pompe à perfusion peut alors être configurée, programmée et la perfusion démarrée, ceci indépendamment de la première pompe.

Le fonctionnement simultané des deux blocs-pompes est illustré sur la figure 9. En supposant qu'une perfusion est en cours (252) sur l'unité 1 ou premier bloc-pompe, les paramètres de perfusion de l'unité 1 sont accessibles (254). L'action sur la touche "écran" permet alors d'avoir accès à l'unité 2 ou deuxième bloc-pompe, et donc aux paramètres de l'unité 2. Plusieurs procédures peuvent alors être mise en oeuvre (260) sur l'unité 2, telles que la configuration, la programmation, le démarrage ou la visualisation des paramètres (262). Un nouvel enfoncement de la touche écran (264) provoque alors l'accès à nouveau aux paramètres de l'unité 1. Durant la phase accès à l'unité 2, la perfusion de l'unité 1 s'est poursuivie normalement. On voit donc que le système se comporte comme deux pompes de perfusion indépendantes se partageant des ressources communes (l'unité de commande, son clavier, son écran)

On doit noter que l'accès à l'autre unité n'est pas autorisée lorsque la première unité se trouve engagée dans certaines procédures telle que les procédures de configuration, de programmation ou de purge.

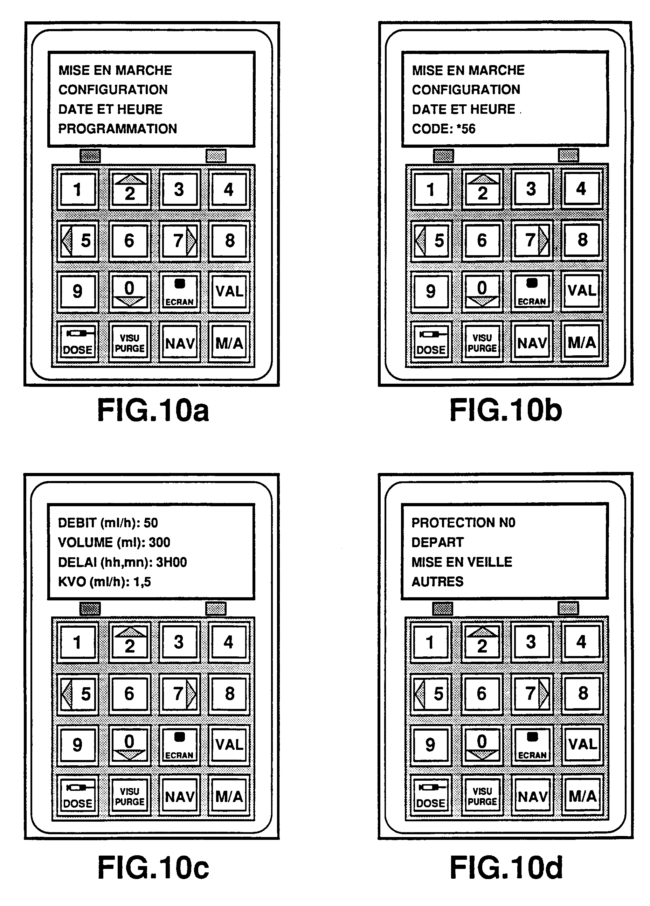

Un exemple de déroulement de procédure est illustré sur les figures 10a-10d. L'exemple illustré concerne la procédure de programmation, mais les autres procédures pouvant être mises en jeu avec l'unité de commande de l'invention sont déroulées d'une façon similaire.

Au départ, l'unité de commande mise sous tension se présente telle qu'illustrée sur la figure 10a, c'est à dire que l'écran affiche les quatre procédures possibles: MISE EN MARCHE, CONFIGURATION, DATE ET HEURE et PROGRAMMATION. A l'aide du curseur haut (la touche 2) du clavier, ou du curseur bas (la touche 0), il est possible de choisir l'une des procédures affichées. En supposant que la procédure PROGRAMMATION soit choisie, le choix est validé par enfoncement de la touche du clavier VAL. Cette validation fait apparaître un nouvel affichage illustré sur la figure 10b où la quatrième ligne est maintenant CODE. Le code d'accès, ici 56, est entré par le clavier et validé par la touche VAL.

Les paramètres de la perfusion apparaissent alors sur l'écran: "Débit", "volume", "délai", "KVO" en face desquels le médecin ou le spécialiste entre les quantités nécessaires. Ainsi, comme illustré sur la figure 10c, le débit est établi à 50 ml/h, le volume de perfusion à 300 ml, le délai à 3 heures. Le KVO (Keep Vein Open) établi à 1,5 ml/h signifie qu'en l'absence d'injection, par exemple dans le mode de configuration INTERMITTENT un débit minimal de 1,5 ml/h est maintenu dans la ligne de perfusion.

Après la validation des paramètres de perfusion, un choix est effectué entre quatre possibilités, comme illustré sur la figure 10d. Il s'agit de DEPART pour démarrer la perfusion, de MISE EN VEILLE qui met la pompe en sommeil, de PROTECTION ou AUTRES. Le choix de PROTECTION N° correspond à différents niveaux dans lesquels le patient est habilité à effectuer certaines opérations. Ainsi, le niveau 0 peut correspondre au cas où le patient ne peut rien faire, le niveau 1 au cas où le patient peut changer lui-même la cassette...etc.

Comme représenté sur les figures 10a à 10d, certaines touches du clavier sont spécialisées. Ainsi, la touche "écran" sert de commutateur pour passer à la visualisation des paramètres de perfusion de l'unité 2 lorsque l'unité visualisait auparavant les paramètres de l'unité 1. La touche PURGE sert à la purge d'air de la ligne de perfusion. La touche DOSE est utilisée directement par le patient en mode PCA pour procéder à l'injection d'une dose d'analgésique à intervalles réguliers. Mais il est évident que d'autres fonctions peuvent être assignées à ces touches du clavier, ou que d'autres touches peuvent avoir des fonctions spécifiques non représentées sur les figures 10a - 10d.

Bien que dans le mode de réalisation préféré de l'invention, la commande du moteur du bloc-pompe soit réalisée par des connexions physiques, il est possible de concevoir une télécommande. Dans ce cas, l'unité de commande disposera d'un émetteur de signaux et le bloc-pompe d'un récepteur mais également d'une pile d'alimentation chargée de fournir l'énergie du moteur qu'il ne reçoit plus de l'unité de commande du fait de l'absence de connexion physique.

## Revendications

1. Système de pompe à perfusion programmable destiné à injecter automatiquement une substance médicamenteuse à un patient, comprenant une ligne de perfusion reliée au corps d'un patient, un premier ensemble composé d'un premier réservoir (14) contenant la substance médicamenteuse et d'un premier bloc-pompe (12) comportant un moteur et une pompe entraînée par le moteur adaptée pour injecter la substance médicamenteuse en provenance du réservoir dans la ligne de perfusion, et une unité de commande programmable (10) destinée à commander le fonctionnement du moteur, ledit ensemble étant détachable de ladite unité de commande tout en restant sous la commande de celle-ci;
ledit système étant caractérisé en ce que ladite unité de commande est de taille réduite, de manière à être portée en permanence par le patient ce qui rend le système ambulatoire, ladite unité de commande comprenant un microprocesseur, un clavier (16) et un écran (18), et ledit microprocesseur étant programmé directement sur le patient à l'aide du clavier pour commander le fonctionnement du moteur selon un programme de perfusion prédéterminé et fournissant des informations sur la perfusion par affichage sur ledit écran.

2. Système selon la revendication 1, caractérisé en ce qu'il comporte en outre au moins un deuxième ensemble comprenant un deuxième bloc-pompe (42) et un deuxième réservoir (44) connecté à ladite unité de commande (10), ledit deuxième ensemble étant détachable de ladite unité de commande tout en maintenant ledit deuxième bloc-pompe sous la commande de celle-ci, de sorte que ladite unité de commande peut commander plusieurs perfusions simultanément.

3. Système selon la revendication 2, caractérisé en ce que chacun desdits ensembles est détachable de ladite unité de commande (10) tout en restant relié à ladite unité de commande par un câble (40) assurant l'alimentation en énergie du moteur dudit ensemble, la transmission des commandes et le transfert des données.

4. Système selon la revendication 2, caractérisé en ce qu'au moins un desdits ensembles comporte une pile d'alimentation pour assurer l'énergie de fonctionnement du moteur, et ladite unité de commande (10) comporte un dispositif de télécommande de telle sorte qu'au moins un desdits ensembles peut être détaché de ladite unité de commande et commandé à distance par cette dernière au moyen dudit dispositif de télécommande.

5. Système selon l'une quelconque des revendications 1 à 4, caractérisé en ce que ladite unité de commande (10) est programmable en fonction du temps absolu de manière à fournir une chronothérapie adaptable au rythme biologique du patient.

6. Système selon l'une quelconque des revendications précédentes, caractérisé en ce que ladite unité de commande (10) comporte une mémoire pour enregistrer l'historique de fonctionnement durant chaque perfusion.

7. Système selon l'une quelconque des revendications précédentes, caractérisé en ce que ladite unité de commande (10) comporte une mémoire pour enregistrer au préalable à toute perfusion des paramètres de contrôle concernant le patient et modifier les commandes fournies par ladite unité de commande (10) lorsque l'un desdits paramètres de contrôle n'est pas respecté lors de la perfusion.

8. Système selon l'une quelconque des revendications précédentes, caractérisé en ce que la partie de la ligne de perfusion qui se trouve dans chacun desdits blocs-pompes est munie d'un capteur de pression (102, 104) chargé de fournir la pression régnant dans cette partie de la ligne soumise à l'action de la pompe, à ladite unité de commande (10).

9. Système selon l'une quelconque des revendications précédentes, caractérisé en ce que des capteurs dans ou sur le corps du patient envoient des données physiologiques à ladite unité de commande (10) de façon à ce que cette dernière puisse commander les paramètres de perfusion en fonction des données reçues.

10. Système selon l'une quelconque des revendications 1 à 9, caractérisé en ce que ladite unité de commande (10) dispose d'un dispositif de lecture de codes à barres de façon à pouvoir être programmée par un stylet optique.

11. Système selon l'une quelconque des revendications 1 à 9, caractérisé en ce que ladite unité de commande (10) dispose d'un dispositif de lecture de carte à puce de façon à pouvoir être programmée par une carte à puce.

12. Système selon l'une quelconque des revendications précédentes, caractérisé en ce que ladite unité de commande (10) peut être connectée à un microordinateur de façon à stocker les données enregistrées dans la mémoire de ladite unité de commande à des fins statistiques, et également de façon à programmer ladite unité de commande.

13. Système selon l'une quelconque des revendications précédentes, caractérisé en ce que ladite unité de commande (10) dispose d'un modem pour pouvoir être branchée sur le réseau téléphonique de façon à ce qu'un médecin ou organisme habilité puisse recueillir les données enregistrées dans la mémoire de ladite unité de commande et exercer ainsi une surveillance à distance.

14. Système selon l'une quelconque des revendications précédentes, caractérisé en ce que ledit réservoir (14) est jetable et est connecté de façon amovible sur ledit bloc-pompe (12) par un dispositif de liaison à enclenchement élastique (50, 52).

## Claims

1. A programmable portable infusion pump system designed for automatically injecting a medicinal substance to a patient, comprising an infusion tube connected to the body of the patient, a first assembly composed of a first supply chamber (14) containing the medicinal substance and a first pump-unit (12) comprising a motor and a pump driven by the motor and designed for injecting the medicinal substance from the supply chamber into the infusion tube, and a programmable controller (10) designed to control the motor, said assembly being detachable from said controller even though remaining under its control;
said system being characterised in that said controller has a reduced size in order to be permanently worn by the patient, which makes the system ambulatory, said controller including a microprocessor, a keyboard (16) and a screen (18), and said microprocessor being directly programmed on the patient thru the keyboard to control the operation of the motor according to a predetermined infusion program and providing information regarding the infusion process thru displaying on the screen.

2. The system according to claim 1, characterised in that it further comprises at least a second assembly including a second pump-unit (42) and a second supply chamber (44), connected to said controller (10), said second assembly being detachable from said controller with said second pump-unit remaining under the control of said controller, so that the controller can control several simultaneous infusions.

3. The system according to claim 2, characterised in that each one of said assemblies is detachable from said controller (10) even though connected to said controller thru a cable (40) supplying power to the motor of said assembly, control transmission and data transfer.

4. The system according to claim 2, characterised in that at least one of said assemblies comprises a battery for powering the motor, and said controller (10) comprises a remote control device so that at least one of said assemblies can be detached from said controller and remotely controlled by the latter thru said remote control device.

5. The system according to any one of claims 1 to 4, characterised in that said controller (10) is programmable relative to absolute time so as to provide a chronotherapy adjustable to the biological pace of the patient.

6. The system according to any one of the preceding claims, characterised in that said controller (10) comprises a memory for storing the history of each ongoing infusion.

7. The system according to any one of the preceding claims, characterised in that said controller (10) comprises a memory to record prior to any infusion control parameters related to the patient, and to modify the commands provided by said controller (10) when one of said parameters is not met during an infusion.

8. The system according to any one of the preceding claims, characterised in that the portion of the infusion tube located in each of said pump-units is equipped with a pressure sensor (102, 104) designed for providing said controller (10) with the measuring of the pressure within the portion of the infusion tube under pressure from the pump.

9. The system according to any one of the preceding claims, characterised in that sensors placed in or on the patient's body transmit to said controller (10) physiological data so that the latter can program the infusion parameters according to the received data.

10. The system according to any one of claims 1 to 9, characterised in that said controller (10) comprises a bar code reading device so as to be programmed by means of an optical pen.

11. The system according to any one of claims 1 to 9, characterised in that said controller (10) comprises an electronic card reader so as to be programmed by means of an electronic card.

12. The system according to any one of the preceding claims, characterised in that said controller (10) can be connected to a personal computer so as to store data recorded in the memory of said controller for statistics purpose, and also so as to program said controller.

13. The system according to any one of the preceding claims, characterised in that said controller (10) is equipped with a modem so as to be connected to the telephone network so that a doctor or qualified organisation can collect data recorded in the memory of said controller and thus remotely monitor the treatment.

14. The system according to any one of the preceding claims, characterised in that said supply chamber can be thrown away and is connected in a removable fashion to said pump-unit (12), thru an elastic clipping mechanism (50, 52).

## Patentansprüche

1. Programmierbares Infusionspumpensystem, das dazu bestimmt ist, einem Patienten automatisch eine Arzneimittelsubstanz zu injizieren, und folgendes aufweist: eine mit dem Körper eines Patienten verbundene Infusionsleitung, eine erste Gruppe, die aus einem die Arzneimittelsubstanz enthaltenden ersten Behälter (14) und einem ersten Pumpenblock (12) besteht, der einen Motor und eine durch den Motor angetriebene Pumpe aufweist, die die vom Behälter kommende Arzneimittelsubstanz in die Infusionsleitung injizieren kann, und eine programmierbare Steuereinheit (10), die dazu bestimmt ist, den Betrieb des Motors zu steuern, wobei diese Gruppe von der Steuereinheit trennbar ist und gleichzeitig unter deren Steuerung bleibt,
dadurch gekennzeichnet, daß die Steuereinheit von geringer Größe ist, so daß der Patient sie ständig bei sich tragen kann, was das System zur ambulanten Behandlung einsetzbar macht, wobei die Steuereinheit einen Mikroprozessor, eine Tastatur (16) und einen Bildschirm (18) aufweist und der Mikroprozessor mit Hilfe der Tastatur direkt an dem Patienten programmiert wird, um den Betrieb des Motors nach einem vorbestimmten Infusionsprogramm zu steuern, und Informationen über die Infusion durch Anzeige auf dem Bildschirm liefert.

2. System nach Anspruch 1, dadurch gekennzeichnet, daß es außerdem mindestens eine zweite Gruppe aufweist, die einen zweiten Pumpenblock (42) und einen zweiten, an die Steuereinheit (10) angeschlossenen Behälter (44) besitzt und von der Steuereinheit trennbar ist, wobei sie gleichzeitzig den zweiten Pumpenblock unter deren Steuerung hält, so daß die Steuereinheit mehrere Infusionen gleichzeitig steuern kann.

3. System nach Anspruch 2, dadurch gekennzeichnet, daß jede dieser Gruppen von der Steuereinheit (10) trennbar ist, wobei sie gleichzeitig mit der Steuereinheit durch ein Kabel (40) verbunden bleibt, das die Energieversorgung des Motors dieser Gruppe und die Übertragung der Steuerungen und der Daten gewährleistet.

4. System nach Anspruch 2, dadurch gekennzeichnet, daß mindestens eine der Gruppen eine Versorgungsbatterie zur Lieferung der Betriebsenergie des Motors besitzt und die Steuereinheit (10) eine Fernsteuervorrichtung aufweist, so daß mindestens eine dieser Gruppen von der Steuereinheit getrennt werden kann und durch diese über die Fernsteuervorrichtung ferngesteuert werden kann.

5. System nach einem der Ansprüche 1 bis 4, dadurch gekennzeichnet, daß die Steuereinheit (10) in Abhängigkeit von der absoluten Zeit programmierbar ist, so daß eine an den biologischen Rhythmus des Patienten anpaßbare Chronotherapie geliefert wird.

6. System nach einem der vorhergehenden Ansprüche, dadurch gekennzeichnet, daß die Steuereinheit (10) einen Speicher zur Aufzeichnung der Betriebsübersicht während jeder Infusion besitzt.

7. System nach einem der vorhergehenden Ansprüche, dadurch gekennzeichnet, daß die Steuereinheit (10) einen Speicher zum Aufzeichnen von den Patienten betreffenden Kontrollparametern vor jeder Infusion und zum Ändern der von der Steuereinheit (10) gelieferten Steuerungen besitzt, wenn einer der Kontrollparameter bei der Infusion nicht eingehalten wird.

8. System nach einem der vorhergehenden Ansprüche, dadurch gekennzeichnet, daß der Teil der Infusionsleitung, der sich in jedem der Pumpenblöcke befindet, mit einem Druckfühler (102, 104) versehen ist, der die Aufgabe hat, den Druck, der in diesem der Wirkung der Pumpe ausgesetzten Teil der Leitung herrscht, der Steuereinheit (10) zu liefern.

9. System nach einem der vorhergehenden Ansprüche, dadurch gekennzeichnet, daß Fühler in oder auf dem Körper des Patienten der Steuereinheit (10) physiologische Daten liefern, so daß diese die Infusionsparameter in Abhängigkeit von den erhaltenen Daten steuern kann.

10. System nach einem der Ansprüche 1 bis 9, dadurch gekennzeichnet, daß die Steuereinheit (10) über einen Strichkodeleser verfügt, so daß sie durch einen Lichtgriffel programmiert werden kann.

11. System nach einem der Ansprüche 1 bis 9, dadurch gekennzeichnet, daß die Steuereinheit (10) über einen Chipkartenleser verfügt, so daß sie durch eine Chipkarte programmiert werden kann.

12. System nach einem der vorhergehenden Ansprüche, dadurch gekennzeichnet, daß die Steuereinheit (10) zur Speicherung der im Speicher der Steuereinheit aufgezeichneten Daten zu statistischen Zwecken und ferner zur Programmierung der Steuereinheit an einen Mikrorechner angeschlossen werden kann.

13. System nach einem der vorhergehenden Ansprüche, dadurch gekennzeichnet, daß die Steuereinheit (120) über ein Modem verfügt, um an das Fernsprechnetz angeschlossen werden zu können, so daß ein Arzt oder eine zuständige Stelle die im Speicher der Steuereinheit aufgezeichneten Daten empfangen kann und auf diese Weise eine Fernüberwachung vornehmen kann.

14. System nach einem der vorhergehenden Ansprüche, dadurch gekennzeichnet, daß der Behälter (14) ein Einwegbehälter ist und an den Pumpenblock (12) durch eine Verbindungsvorrichtung (50, 52) mit elastischer Einrastung lösbar angeschlossen ist.
